# EUROPÄISCHE PATENTANMELDUNG

(11) **EP 3 574 932 A1**
(43) Veröffentlichungstag der Anmeldung: **04.12.2019**
(21) Anmeldenummer: 18174614.0
(22) Anmeldetag: 28.05.2018
(51) Int. Cl.: A61M 1/10, A61M 1/12

(54) **BLUTPUMPE**

(71) Anmelder: Berlin Heart GmbH, 12247 Berlin (DE)
(72) Erfinder: Richert, Hendryk, 12487 Berlin (DE); Peters, Oliver, 14129 Berlin (DE)
(74) Vertreter: Pfenning, Meinig & Partner mbB

(57) **Zusammenfassung**

Gegenstand der vorliegenden Anmeldung ist eine Pumpe mit einem Gehäuse und einem stromaufwärtigen Einlass und einem stromabwärtigen Auslass und einem zwischen diesem Einlass und dem Auslass angeordneten Fluidkanal mit einer Kanalachse. Innerhalb des Fluidkanals ist ein Rotor angeordnet, welcher mittels eines Motors in Rotation versetzt werden kann.

Ferner ist eine Sensoranordnung vorhanden, welche eine Neigung der Rotationsachse des Rotors erfassen kann.

## Beschreibung

Gegenstand der vorliegenden Anmeldung ist eine Pumpe, vorzugsweise eine Blutpumpe, zum Einsatz in Herzunterstützungssystemen. Insbesondere die Verwendung als "Ventricular Assist Device" (VAD) für das linke Ventrikel (LVAD) oder das rechte Ventrikel (RVAD) oder im Rahmen eines Systems für beide Ventrikel (BiVAD).

Die Überwachung des Flusses des gepumpten Fluids oder anderen von Pumpenparametern ist insbesondere bei Blutpumpen von großer Wichtigkeit. Um die Pumpleistung von Pumpen genau einstellen zu können, ist entweder eine Überwachung von Pumpenparametern mittels Sensoren oder eine Schätzung der Parameter und eine entsprechende Auswertung von Nöten.

Ein wichtiger Parameter ist die Schätzung bzw. die Messung des durch die Pumpe geförderten Flusses. Bei Blutpumpen ist die Flussschätzung bzw. Flussmessung eine Voraussetzung für die physiologische Regelung in einer Pumpe. Als Flusssensor können beispielsweise Ultraschallsensoren oder Ultraschallmessungen eingesetzt werden. Bei aktiv magnetisch gelagerten Pumpen, wie beispielsweise der HeartMate III von Thoratec, wird eine Positionsänderung des Rotors gegenüber des Gehäuses innerhalb einer Ebene quer zu einer Einlassachse zur Flussschätzung verwendet.

In der WO 2017/015268A1 wird eine Flussschätzung anhand der Auslenkung eines Pumpenrotors bei einer magnetisch aktiv gelagerten Radialpumpe mittels Halleffektsensoren beschrieben. Die Rotorauslenkung muss permanent überwacht werden, um den Betrieb der Pumpe gewährleisten zu können. Die Regelung von aktiv gelagerten Pumpen ist aufwendig und erfordert zahlreiche Kontrollmechanismen.

In der WO 2009/132707A1 wird ein Verfahren zur Bestimmung einer Verkippung eines Rotors einer Rotationsmaschine beschrieben, mithilfe dessen ein magnetisch aktiv gelagerter Motor bzw. dessen Position zwischen den beiden Vor- und Nachleiträdern bestimmt wird. Auch hier handelt es sich um eine aktiv gelagerte Pumpe, deren Überwachung aufwendig ist.

Aufgabe der vorliegenden Anmeldung ist es eine alternative Blutpumpe bereitzustellen, welche einen weniger komplexen Aufbau besitzt. Ferner wird ein Verfahren vorgeschlagen eine derartige Pumpe für eine Flussschätzung zu verwenden.

Die Aufgabe wird durch die Pumpe gemäß Anspruch 1 gelöst. Alternative oder zusätzliche Merkmale der Pumpe finden sich in den abhängigen Ansprüchen und den nachstehenden Ausführungen.

Die Pumpe umfasst ein Gehäuse mit einem stromaufwärtigen Einlass und einem stromabwärtigen Auslass. Zwischen dem Einlass und dem Auslass ist ein Fluidkanal mit einer ersten Kanalachse vorhanden, entlang dem das zu fördernde Fluid vom Einlass in Richtung des Auslasses transportiert wird. Stromabwärts des Einlasses befindet sich ein Rotor mit einem stromaufwärtigen und einem stromabwärtigen Ende, wobei der Rotor eine Beschaufelung zur Förderung des Fluids aufweist. An seinem stromaufwärtigen Ende ist der Rotor durch ein mechanisches oder hydrodynamisches Lager gelagert.

Im Falle eines mechanischen Lagers kann stromaufwärts des Rotors ein Vorleitrad im oder außerhalb des Fluidkanal(s) angeordnet werden, wobei das Vorleitrad mittels Streben, welche das Vorleitrad halten, oder durch Flügel, welche das Vorleitrad halten und dem zu fördernden Fluid eine signifikante Drallströmung aufzwingen, gehalten wird. Am stromaufwärtigen Ende des Rotors kann beispielsweise ein Lagerkopf angeordnet sein, im Vorleitrad kann eine dazu korrespondierende Lagerkalotte (oder vice versa) angeordnet sein. Details zu entsprechenden Lagern kann beispielsweise den Anmeldungen EP 18 164 553.2 und PCT/EP2017/074796 entnommen werden, welche vollumfänglich zum Bestandteil dieser Anmeldung gemacht werden.

Im Falle eines hydrodynamischen Lagers kann stromaufwärts des Rotors eine Fluidkanalverengung angeordnet werden, sodass sich in der Richtung der Kanalachse zwischen dem Rotor und der Fluidkanalverengung ein hydrodynamisches Lager ausbildet.

Der Teil des Fluidkanals, in welchem der Rotor angeordnet ist, umfasst einen rohrförmigen Abschnitt mit einem im Wesentlichen kreisrunden Querschnitt. Der Fluidkanal kann ferner, sich an den rohrförmigen Abschnitt anschließend, eine Schnecken-, eine Spiral-, oder eine Ringkammer umfassen, in welcher der Auslass angeordnet ist. Der Rotor kann sich dabei aus dem Rohr bis in die Ring-, Spiral- oder Schneckenkammer erstrecken oder kann ausschließlich im Rohr förmigen Abschnitt angeordnet sein.

Bei der anmeldungsgemäßen Pumpe handelt es sich um eine Axialmaschine, d. h. dass der Rotor das Blut axial fördert und einen Druckaufbau initiiert und das Fluid anhand des Druckgefälles aus der Pumpe gepumpt wird. Als Beschaufelung bietet sich hierbei in zahlreichen Varianten eine wendelartige Beschaufelung an. Die Wendel kann dabei weniger als einen vollen Umlauf um den Rotorkörper, einen gesamten Umlauf um den Rotorkörper, oder mehr als einen Umlauf um den Rotorkörper besitzen in zahlreichen Ausführungsbeispielen besitzt der Rotor mehr als eine Wendel, beispielsweise 2 oder 3 Wendel. Die Steigung zwischen den Wendeln nimmt dabei vom stromaufwärtigen Ende des Rotors zum stromabwärtigen Ende des Rotors hin zu. In zahlreichen Ausführungsbeispielen erstreckt sich die Beschaufelung vom stromaufwärtigen Ende des Rotors bis zur Hälfte des Rotors, in anderen Ausführungsbeispielen kann sich die Beschaufelung weiter zum stromabwärtigen Ende hin erstrecken.

Der Rotor wird mittels eines im Gehäuse angeordneten Motors in Rotation versetzt. Der Motorstator umläuft einen innerhalb des Rotors angeordneten Motorrotor und treibt diesen an.

Wie eingangs erwähnt, wird das stromaufwärtigen Ende des Rotors mittels eines mechanischen Lagers oder eines hydrodynamisches Lagers gehalten. Zusätzlich zu diesem Lager besitzt die Pumpe ein passives magnetisches Lager, mithilfe dessen der stromabwärtige Teil des Rotors in der gewünschten Position gehalten wird. Bevorzugt handelt es sich hierbei um ein passives magnetisches Radiallager, welches den Rotor radial lagert, axial vorspannt und auf das mechanische oder hydrodynamische Lager drückt. Eine Komponente des passiven magnetischen Lagers ist im Rotor angeordnet und kann beispielsweise ein magnetischer Ring oder Zylinder, oder mehrere Ringe/Zylinder in Squeeze-Field- oder Halbachanordnung sein. Das Gegenstück hierzu ist im Gehäuse angeordnet und kann in einigen Ausführungsbeispielen den im Rotor angeordneten Ring umlaufen. Um eine Vorspannung zu erreichen, können die im Gehäuse und Rotor angeordneten Magnetlagerkomponenten axial entlang der Kanalachse zueinander versetzt sein. Alternativ oder zusätzlich zum passiven magnetischen Lager, kann ein hydrodynamisches Lager oder ein flexibles mechanisches Lager vorhanden sein.

Ferner umfasst die Pumpe eine Sensoranordnung zur Erfassung einer Neigung der Rotationsachse des Rotors. Die Sensoranordnung kann dabei eine Abweichung der Rotationsachse von der Kanalachse erfassen. Der Fluidkanal bildet ein festes Koordinatensystem in dem die Kanalachse festliegt, und beispielsweise die z-Achse eines kartesischen Koordinatensystems bildet. Der Rotor welcher um die Rotationsachse rotiert kann im laufenden Betrieb der Pumpe, d. h. während der Rotor durch den Motor angetrieben wird eine Auslenkung aus der z-Achse erfahren. So kann beispielsweise der zu fördernde Fluss eine Verkippung des Rotors um den Auflagepunkt im Bereich des mechanischen bzw. hydrodynamischen Lagers bewirken. Diese Auslenkung kann erfasst und als Schätzung der Druckdifferenz in der Volute und für den geförderten Fluss verwendet werden. Ferner kann die Verkippung des Rotors, d. h. die Abweichung der Rotationsachse von der Kanalachse zeitaufgelöst erfasst werden und anhand der Frequenzen der Bewegung des Rotors können beispielsweise die Änderung der Turbulenz (Thrombenwachstum, eingeschwemmte Thromben) oder andere physiologische Aktivitäten (Puls, Atmung, Bewegung) sowie Unregelmäßigkeiten erfasst werden.

Da es sich bei der Pumpe um eine passiv gelagerte Pumpe handelt welche in einigen Ausführungsbeispielen keine aktive Regelung der Lagerung besitzt, ist der Aufbau der Pumpe sehr einfach. Die Sensoranordnung wird lediglich verwendet um die Verkippung des Rotors gegenüber dem Gehäuse zu ermitteln und muss keine sicherheitsrelevante Funktion erfüllen. Jedoch kann die Verkippung auch verwendet werden, um die Patientensicherheit zu erhöhen. Lediglich die Umdrehungsgeschwindigkeit des Motors muss aktiv geregelt werden.

Bevorzugt handelt es sich bei der Sensoranordnung um eine Sensoranordnung, welche ein durch eine Komponente des Rotors oder durch den Rotor selbst erzeugtes Magnetfeld erfasst und auswertet Dabei ist bevorzugt dass die Sensoranordnung symmetrisch um die Kanalachse angeordnet ist. D. h. entweder ist ein Sensor entlang der Kanalachse angeordnet, oder eine Vielzahl von Sensoren ist in einer Ebene senkrecht zur Kanalachse um diese herum, bevorzugt symmetrisch angeordnet.

Alternativ oder zusätzlich kann die Sensoranordnung stromabwärts des Rotors angeordnet sein. Bei einer Anordnung stromabwärts des Rotors ist es auf einfache Art und Weise möglich ein in Richtung der Kanalachse verlaufendes Magnetfeld des Rotors zur Auswertung der Verkippung des Rotors zu verwenden. Da die Sensoranordnung bevorzugt in einer Ebene quer zur Kanalachse angeordnet ist wird die Richtung des verlaufenden Magnetfelds des Rotors genau messbar und somit die Genauigkeit der Fassung der Verkippung des Rotors sehr hoch. Bevorzugt werden in der Sensoranordnung mindestens ein magnetoresistiven Sensor (z. B. einen anisotropen magnetoresistiven (AMR), giant magnetoresistiven (GMR) oder tunnel magnetoresistiven (TMR) Sensor), welche eine Magnetfeldstärke oder -richtung bestimmen kann, oder mehrere Halleffektsensoren, wie beispielsweise drei oder mehr Halleffektsensoren in einer derartigen Sensoranordnung verwendet. Möglich ist auch der Einsatz von Fluxgate- oder magnetoindukiver Sensorik. Im Falle eines magneto-resistiven Sensors ist lediglich genau ein Sensor von Nöten (es können jedoch auch mehr als ein Sensor verwendet werden). Dieser eine Sensor erfasst die Richtung des Magnetfelds beispielsweise entlang der Kanalachse, und die Richtung des Magnetfelds innerhalb der Ebene quer zur Kanalachse. Der resultierende Magnetfeldvektor kann verwendet werden, um eine Verkippung des Rotors gegenüber der Kanalachse zu erfassen, da die Richtung des Magnetfelds des Rotors gegenüber der im Ruhezustand verlaufenden Magnetfeldrichtung verschoben ist. Aus dieser Magnetfeldrichtungsverschiebung kann die Verkippung des Rotors ermittelt werden. Im Falle mehrerer Halleffektsensoren kann beispielsweise ein Sensorarray von drei oder mehr Sensoren innerhalb einer (bevorzugt einer einzigen) Ebene quer zur Kanalachse eingesetzt werden. Die einzelnen Halleffektsensoren erfassen dabei die Magnetfeldstärke entlang der Richtung der Kanalachse. Aus der Kombination der Signale der mehreren Halleffektsensoren kann eine Verkippung des Rotors ermittelt werden, da mit einer Verkippung des Rotors eine Verkippung der Magnetfeldrichtung, welche durch den Rotor erzeugt wird einhergeht. Alternativ oder zusätzlich zu den bereits oben erwähnten Sensortypen können auch Fluxgatesensoren oder Induktionsspulen oder mit Magneten belastete Kraftsensoren verwendet werden.

Alternativ oder zusätzlich kann die Sensoranordnung derart ausgebildet sein dass sie zum differentiellen Messen des Magnetfelds ausgebildet ist. Hierbei werden Sensoren jeweils paarweise in einer Ebene quer zur Kanalachse angeordnet, sodass eine Verkippung in Richtung des ersten Sensors des Sensorpaares bei diesem ersten Sensor zu einer Magnetfelderhöhung führt, wohingegen der zweite Sensor eine Magnetfeld Schwächung erfährt. Mittels des differentiellen Messens des Magnetfelds kann eine besonders genaue Erfassung des Magnetfelds bewirkt werden. Die differentielle Messung unterdrückt zudem örtlich gleichförmige Störfelder wie zum Beispiel das Erdmagnetfeld.

Alternativ oder zusätzlich umfasst der Rotor einen magnetischen Flusskonzentrator, welcher vorzugsweise im Bereich des stromabwärtigen Endes des Rotors angeordnet ist. Sofern die Sensoranordnung eine Sensoranordnung zur Erfassung eines Magnetfelds ist, kann ein Flusskonzentrator, beispielsweise aus Weicheisen, den magnetischen Fluss in Richtung der Kanalachse erhöhen und so die Messgenauigkeit verbessern. Alternativ oder zusätzlich kann der Rotor stromaufwärts des Flusskonzentrators einen Flussgenerator aufweisen, um das zu konzentrieren Magnetfeld in Rotor zu erzeugen. Als Flussgenerator bietet sich hierbei insbesondere eine Komponente des passiven Magnetlagers an, welche im Rotor angeordnet ist. Dabei ist der Flussgenerator vorzugsweise ein magnetischer Ring oder eine magnetische Scheibe/Zylinder, welche jeweils um die Rotationsachse des Rotors angeordnet ist.

Alternativ oder zusätzlich verjüngt sich der Rotor zu seinem stromabwärtigen Ende hin. Durch die Verjüngung können zum einen strömungstechnische Vorteile bei der Förderung des Fluids bewirkt werden, und zum anderen eine Konzentration des Magnetfelds in Bereich des verjüngten Endes erreicht werden. Hierdurch wird die Magnetfeldstärke entlang der Rotationsachse erhöht, was wiederum die Messgenauigkeit erhöht. In einer Variante kann der Rotor beispielsweise eine Tropfenform aufweisen. In anderen Varianten kann der Rotor an seinem Strom abfertigen Ende beispielsweise kegelstumpf-artig oder kegelartig zu laufen.

Alternativ oder zusätzlich ist am Gehäuse ein mechanisches oder hydrodynamisches Fanglager angeordnet, welches das stromabwärtige Ende des Rotors in seiner Bewegungsfreiheit einschränkt. Das Fanglager kann beispielsweise derart angeordnet sein, dass der Rotor bei einer zu großen Verkippung mechanisch oder hydrodynamisch blockiert wird. Das mechanische Fanglager kann beispielsweise als aus dem stromabwärtigen Teil des Gehäuses herausragender Ring beschaffen sein, an welchen das stromabwärtige Ende des Rotors bei einer zu starken Verkippung anschlägt. Hierdurch wird ein sicherer Betrieb der Pumpe gewährleistet.

Wie bereits eingangs erwähnt, kann der Fluidkanal alternativ oder zusätzlich in eine Auslasskammer übergehen, wobei die Auslassachse des Auslasses gegenüber der Kanalachse um einen Winkel geneigt ist, vorzugsweise im Wesentlichen senkrecht dazu verläuft. Durch den tangentialen Auslass wird eine Verkürzung der Pumpe bewirkt und der Arbeitsbereich der Pumpe positiv beeinflusst. Dabei kann in einigen Ausführungsformen vorgesehen sein dass sich die Beschaufelung des Rotors nicht in die Auslasskammer erstreckt sondern lediglich in den rohrförmigen Abschnitt angeordnet ist. Obgleich bislang nur ein einziger Auslass erwähnt wurde, kann die Pumpe weitere Auslässe aufweisen, beispielsweise zwei oder mehr Auslässe.

Wie eingangs bereits beschrieben ist das passive magnetische Lager ein passives magnetisches Radiallager. Dieses kann im Bereich eines rohrförmigen Abschnitts, vorzugsweise stromabwärts eines Motorstators angeordnet sein. Unter einem Radiallager wird hierbei verstanden, dass das Lager radial stabil gelagert ist. Das Lager kann jedoch derart ausgelegt werden, dass es axial instabil ist.

Mittels der Sensoranordnung kann eine Verkippung des Rotors erfasst werden. Hierbei ist es jedoch nicht notwendig die Verkippung des Rotors in Grad zu erfassen, sondern es ist ausreichend die Magnetfeldänderungen zu erfassen. Aus den gemessenen Magnetfeldänderungen kann die Position des stromabwärtigen Endes des Rotors und somit die Verkippung bestimmt werden. Hierbei kann vorgesehen sein, dass anhand von Tabellen, welche im Pumpencontroller abgelegt sind, oder simpler, mehrdimensionaler Approximationen mit Polynomen n-ten Grades (vorzugsweise nicht höher als 4) die Magnetfeldänderung in Kombination mit der Drehzahl und Stromaufnahme des Motors und einer geschätzten Viskosität in eine Druckdifferenz oder/und einen geförderten Flusses übersetzt wird. Ferner kann die Bewegung des Rotors insbesondere dessen Bewegungsfrequenzen dahingehend genutzt werden um im Bereich der Pumpen Thromben zu detektieren und die Viskosität des Blutes zu schätzen. Weiterhin kann die (ggf. zeitaufgelöste) Verkippung ausgewertet werden, um einen Verschleiß des stromaufwärtigen mechanischen Lagers, eines magnetischen Lagers oder der Motorkomponenten zu erkennen. Ferner kann die Verkippung zur Schätzung des Drucks, oder eines zusammengesetzten Kennwerts verwendet werden, wobei der zusammengesetzte Wert sich aus zwei oder mehr der nachstehenden Größen zusammensetzt: Fluss, Druck, Viskosität, Ablagerung und Verschleiß. Alternativ oder zusätzlich ist vorgesehen, dass die Sensoranordnung derart angeordnet ist, dass das Magnetfeld der Motormagnete vernachlässigbar ist, oder gar isoliert ist. Das Motormagnetfeld wird dann nicht zur Detektion herangezogen.

In den nachfolgenden Figuren werden einige Ausführungsbeispiele näher erläutert. Es zeigen:
- Figur 1A: schematische Ansicht einer Axialpumpe mit tangentialem Auslass;
- Figur 1B: schematische Ansicht der Rückseite der in Figur 1A gezeigten Pumpe;
- Figur 2: Längsschnitt durch die in der Figur 1A gezeigten Pumpe (schematisch);
- Figur 3: Magnetfeldverlauf zwischen dem Rotor und dem im Gehäuse angeordneten Magneten
- Figur 4: schematische Darstellung der Sensoranordnung und des Rotors;
- Figuren 5a bis c:: Verkippung eines Rotors sowie dazugehöriger Magnetfeldverlauf und Trajektorie des stromabwärtigen Endes des Rotors;
- Figur 6: alternative Sensoranordnung und schematische Funktionsweise.

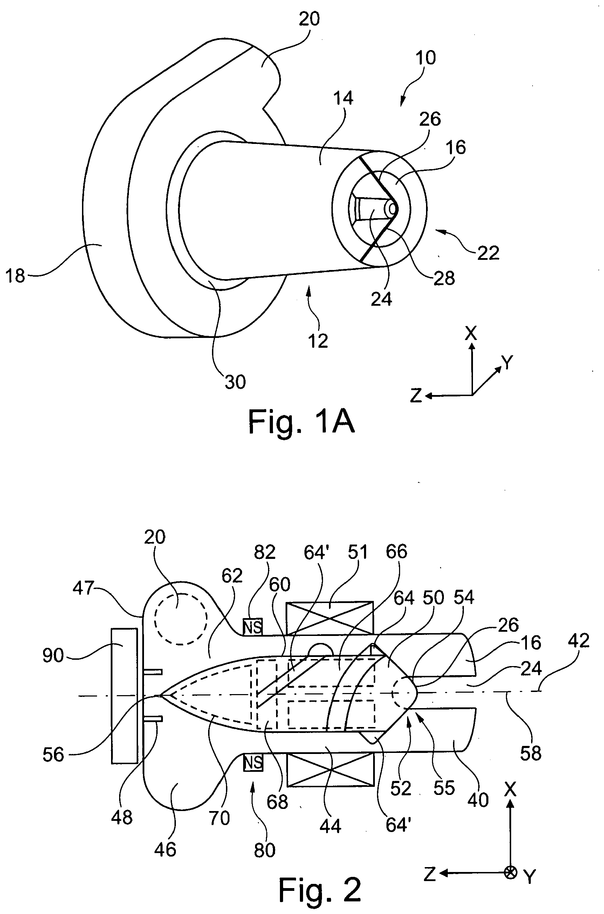

Die in der Figur 1A dargestellte Pumpe 10 umfasst von außen betrachtet ein Gehäuse 12, welches zum einen einen rohrförmigen Abschnitt 14 mit einem Einlass 16 umfasst; und ferner eine Auslasskammer 18 mit einem Auslass 20 besitzt. Am Einlassbereich 22 der Pumpe 10 ist zudem ein Lagerstator 24 erkennbar, welcher mittels Streben 26 und 28 mittig und koaxial zum rohrförmigen Abschnitt 14 gehalten wird. Der Lagerstator 24 bildet den feststehenden Teil eines mechanischen Lagers, auf welches noch näher eingegangen wird. Die Streben 26 und 28 sind dünn gewählt und besitzen einen derartigen Querschnitt, das diese ein einströmendes Fluid in seinem Strömungsverhalten nicht wesentlich ändern. Beispielsweise können die Streben eine in der xy-Ebene gemessene Dicke von 0.5-3 mm, eine in der z-Richtung gemessene Länge von 2-10 mm und ein im Wesentlichen ovales oder kreisrundes Profil besitzen. Im Wesentlichen verlaufen die Streben entlang der z-Richtung

Alternativ hierzu kann der Lagerstator 24 beispielsweise durch Flügel gehalten werden, welche dem zu förderndem Fluid eine Drallströmung aufzwingen. Die Flügel besitzen in einigen Ausführungen eine Krümmung (ähnlich einer Rotorbeschaufelung), um dem Fluid eine Pre-Rotation aufzuzwängen. Die Flügel können eine Länge von bis zu 15 mm besitzen. Im Stand der Technik wird auch häufig von einem Vorleitrad gesprochen.

Der Auslass 20 ist derart konfiguriert, dass dieser beispielsweise mittels eines Konnektors mit einem Graft oder eine Silikonkanüle verbunden werden kann. An der Pumpe ist zudem ein Nahtringkonnektor 30 erkennbar, mittels welchem die Pumpe an einem Nahtring, welcher am Herz angeordnet ist, befestigbar ist. Der rohrförmige Abschnitt 14 des Gehäuses 12 wird dabei in eine Öffnung, beispielsweise des linken Ventrikels geschoben, wohingegen ein mit dem Auslass verbundener Graft oder einer Auslasskanüle mit der Aorta oder beispielsweise der Subklaviarterie verbunden wird.

Die hier dargestellte Pumpe 10 ist eine Axialmaschine mit einem tangentialen Auslass 20, d.h. dass der Rotor das Blut in axialer Richtung fördert und den Druck des Fluids stromabwärts erhöht, um das Fluid zu fördern. In anderen Ausführungsbeispielen kann die Pumpe jedoch eine Radialmaschine sein, bei welcher der Rotor das Blut nach Außen beschleunigt.

Bei der Auslasskammer 18 kann es sich um eine Ringkammer, welche gegenüber dem Fluidkanal des rohrförmigen Abschnitts 14 keine radiale Aufweitung besitzt; um eine Spiralkammer, welche eine radiale Aufweitung der Auslasskammer gegenüber dem Fluidkanal des rohrförmigen Abschnitts aufweist; oder um eine Schneckenkammer, welche sowohl eine radiale als auch eine axiale Aufweitung des Fluidkanals des rohrförmigen Abschnitts zum Auslass 20 hin besitzt, handeln.

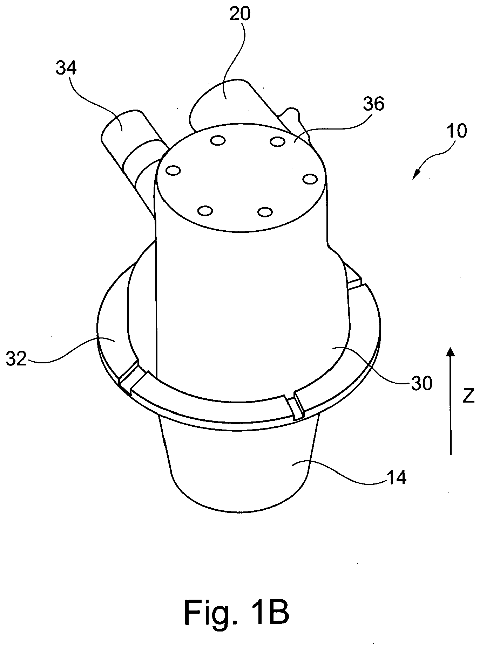

In der Figur 1B ist die in der Figur 1A dargestellte Pumpe 10 in einer anderen Ansicht gezeigt. Auch hier ist der rohrförmige Abschnitt 14 zu sehen. Ferner ist ein zum Nahtringkonnektor 30 korrespondierender Nahtring 32 eingezeichnet, welcher im implantierten Zustand mit dem Ventrikel des Herzens verbunden ist. Der Auslass 20 ist gut erkennbar. Neben dem Auslass 20 ist zudem ein Ausgang für die Driveline 34 erkennbar. Mittels der Driveline werden elektrische Energie zum Betrieb der Pumpe, aber auch Steuersignale an die Pumpe gesendet. In der Pumpe erfasste Signale, welche beispielsweise von der Sensoranordnung empfangen werden, werden an eine externe Kontrolleinheit weitergeleitet werden. In Z-Richtung betrachtet befindet sich an die Auslasskammer anschließend ein Gehäuseteil, welcher als Kammer 36 für die Sensoranordnung dient. Dies bedeutet, dass die Sensoranordnung in der Kammer 36 gehalten ist, die Sensoranordnung jedoch nicht im Fluidkanal liegt.

In der Figur 2 ist ein Längsschnitt entlang der xz-Ebene der Pumpe 10 der Figur 1A dargestellt. Zwischen dem Einlass 16 und dem Auslass 20 erstreckt sich ein Fluidkanal 40 mit einer Kanalachse 42, welche hier derart gewählt ist, dass sie entlang der z-Achse verläuft. Im Fluidkanal ist ein Rotor 50 angeordnet, welcher durch einen nicht im Fluidkanal liegenden Motorstator 51 in Rotation versetzt werden kann. Der Fluidkanal 40 umfasst einen zylinderförmigen Abschnitt 44, welcher in eine Spiralkammer 46 übergeht. Dabei ist erkennbar, dass der Durchmesser der Auslasskammer entlang der x-Achse größer ist als der Durchmesser des zylinderförmigen Abschnitts 44.

Am stromabwärtigen Ende des Fluidkanals 40, im Bereich der Spiralkammerwand 47 befindet sich zudem ein ringförmiger Anschlag 48, welcher als mechanisches Fanglager dient und auf welchem später noch eingegangen wird. Außerhalb des Fluidkanals und innerhalb des Gehäuses befindet sich stromabwärts der stromabwärtigen Wand 47 des Gehäuses 12 eine Sensoranordnung 90, welche ebenfalls noch erläutert werden wird.

In der Figur 2 ist der Lagerstator 24 gut erkennbar. Der Lagerstator 24 endet an seinem stromabwärtigen Ende 26 in einer Lagerkalotte, welche beispielsweise mit einer Hartkeramik auf Carbidbasis oder einem Diamant beschichtet oder ausgebildet sein kann. Beispielhafte Lagerkalotten finden sich beispielsweise in der PCT/EP2017/074796, welche vollumfänglich zum Bestandteil dieser Anmeldung gemacht wird.

Der Rotor 50 weist an seinem stromaufwärtigen Ende 52 eine zur Lagerkalotte 26 korrespondierende Lagerkomponente 54 auf, welche beispielsweise eine mit diamantbeschichtete Kugel oder ein aus Diamant hergestelltes Kugelsegment seien kann. Das aus den Komponenten 54 und 26 gebildete mechanische Lager 55 dient als axial/radial Lager, da der Rotor 50 während der Förderung des Fluids durch den Fluiddruck an die Lagerkalotte 26 gepresst wird. Ferner dient das mechanische Lager 55 als Auflagepunkt für den Rotor 50, um welchen der Rotor 50 selbst kippen kann. Bei einer Verkippung des Rotors aus der Kanalachse heraus, wird das stromabwärtige Ende 56 des Rotors in x- oder y-Richtung verkippt. Während in der Figur 2 die Rotationsachse 58 des Rotors 50 mit der Kanalachse 42 koinzidiert, kann die Rotationsachse 58 im Pumpenbetrieb von der Kanalachse 42 abweichen, sodass die beiden einen Winkel α bilden. Bei dem hier dargestellten Lager 55 aus den Komponenten 26 und 54 bildet dieses den Schnittpunkt zwischen den beiden Geraden.

Der Rotor 50 weitet sich von der Lagerkomponente 54 in z-Richtung auf, nimmt in seinem, in der xy-Ebene gemessenen Ausmaß zu und mündet in einem zylinderförmigen Abschnitt 60. Dieser zylinderförmige Abschnitt 60 verjüngt sich anschließend zu einem konus-, bzw. kegelstumpfförmigen Abschnitt 62. Der Übergang zwischen dem zylinderförmigen Abschnitt 60 und dem kegelstumpfförmigen Abschnitt 62 liegt noch innerhalb des zylinderförmigen Abschnitts 44, kann in anderen Ausführungsformen jedoch genau auf dem Übergang zwischen Abschnitt 44 und Spiralkammer 46, oder in der Spiralkammer 46 beginnen. An seinem stromabwärtigen Ende 56 ist der Rotor 50 derart beschaffen, dass dieser nur einen geringen radialen Querschnitt besitzt, beispielsweise einen Querschnitt von 3-80 mm^2 besitzen. Demgegenüber kann der Rotor im zylinderförmigen Abschnitt einen Querschnitt von 20 - 180 mm^2 besitzen.

An der Außenseite des Rotors 50 befindet sich eine Beschaufelung 64, welche im vorliegenden Ausführungsbeispiel wendelförmig ausgebildet ist. Auf dem Rotor befindet sich eine erste Wendel 64 und eine zweite Wendel 64', welche um 180° zueinander versetzt angeordnet sind. Die Wendeln erstrecken sich dabei um weniger als 360° um den Rotorumfang. Dies ist beispielsweise durch den Verlauf der Wendel 64' erkennbar. Die Steigung (Pitch) zwischen den beiden Wendeln, d.h. der Abstand in axialer Richtung zwischen den Wendeln, nimmt in z-Richtung zu. Weitere Beispiele für die Rotorform bzw. für die Anordnung der Beschaufelung auf einem Rotor finden sich beispielsweise in der EP 18 164 553.2 oder der WO 2011/054545, welche beide vollumfänglich zum Bestandteil dieser Anmeldung gemacht werden.

Der Rotor kann beispielsweise aus Metall, wie z.B. Stahl, Titan, oder anderen bekannten biokompatiblen Materialien aufgebaut sein. Im Inneren des Rotors befinden sich neben einem Motorrotor, welcher beispielhaft als Element 66 eingezeichnet ist, ein in z-Richtung magnetisierter Ring 68, welcher zum einen Teil eines Radiallagers 80 ist, und zum andern einen magnetischen Fluss des Rotors entlang der Rotationsachse 58 generiert. Ferner befindet sich innerhalb des kegelstumpfförmigen Abschnitts 62 des Rotors ein Flusskonzentrator 70, welcher das Magnetfeld des Flussgenerators 68 zur Rotationsachse 58 hin konzentriert und dem Rotor stromabwärts ein Magnetfeld mit ausgeprägter Magnetfeldkomponente in z-Richtung beschert. In einigen Ausführungsbeispielen wird die in z-Richtung wirkende Magnetfeldkomponente des Rotors durch die Sensoranordnung 90 ausgewertet, um eine Verkippung des Rotors, d.h. eine Verkippung der Rotationsachse 58 gegenüber der Kanalachse 42, zu erfassen und auszuwerten.

Wie bereits erwähnt ist der magnetische Ring 68 auch Bestandteil eines passiven Magnetlagers 80. Dieses umfasst ferner einen magnetischen Ring 82, welcher im Gehäuse außerhalb des Fluidkanals 40 angeordnet ist. Der magnetische Ring 82 ist ebenfalls axial polarisiert, d.h. dessen Magnetfeld ist ebenfalls in der z-Richtung ausgerichtet. Die magnetischen Ringe 68 und 62 sind als stabiles Radiallager ausgebildet, sodass der Rotor einen Gleichgewichtszustand besitzt, sofern die Rotationsachse 58 mit der Kanalachse 42 korrespondieren. Ferner sind die beiden Ringe axial zueinander versetzt angeordnet, was eine zusätzliche Vorspannung des Rotors in axialer Richtung bewirkt. Die Ringe 82 und 68 können auch aus radial entgegengesetzt magnetisierten Ringen zusammengesetzt oder als radiale Halbacharrays aufgebaut sein. Die Sensoranordnung wird dann durch den axial verlaufenden magnetischen Streufluss gespeist.

Der Abstand zwischen dem stromabwärtigen Ende 56 des Rotors 50 und der Sensoranordnung 90 kann in der z-Richtung beispielsweise zwischen 0,5 mm und 30 mm betragen. Im vorliegenden Beispiel beträgt der Abstand 15 mm.

Obgleich im vorliegenden Ausführungsbeispiel der Rotor 50 stromaufwärts durch ein mechanisches Lager gehalten wird, kann das stromaufwärtige mechanische Lager in anderen Ausführungsbeispielen durch ein hydrodynamisches Lager ersetzt sein. Der Auflagepunkt zur Bestimmung der Verkippung wäre im Falle eines hydrodynamischen Lagers durch einen innerhalb des Rotors sitzenden virtuellen Auflagepunkt gegeben.

Eine Besonderheit der hier vorgestellten Pumpe ist die freie Lagerung des stromabwärtigen Endes des Rotors 50. Wie bereits erwähnt, wird das stromabwärtige Ende lediglich durch ein Fanglager in seiner Bewegung eingeschränkt. Dabei kann das Fanglager derart beschaffen sein, dass lediglich pathologische Pumpenzustände verhindert werden, d.h. beispielsweise ein Anschlagen der Beschaufelung an der Gehäusewand des rohrförmigen Abschnitts verhindert wird. Ansonsten kann das stromabwärtige Ende 56 innerhalb des Rings 48 freie Trajektorien beschreiben.

Die anhand der Sensoranordnung 90 aufgenommenen Signale können beispielsweise durch einen in der Pumpe angeordneten Kontrollschaltkreis (nicht dargestellt) ausgewertet werden oder mittels einer Datenleitung, welche beispielsweise in die Driveline integriert ist, durch einen externen Pumpencontroller ausgewertet werden.

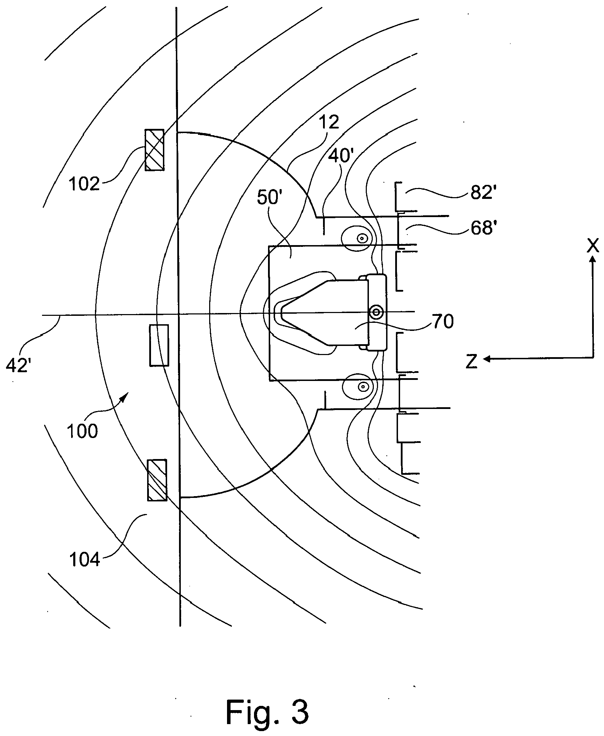

Anhand der Figur 3 soll das in z-Richtung wirkende Magnetfeld der in den Figuren 1 und 2 dargestellten Rotoren schematisch dargestellt werden. In der Figur 3 wird lediglich ein schematischer Fluidkanal 40' dargestellt ohne auf die Auslasskammer näher einzugehen. Gegenständlich ist das Gehäuse 12, in welchem der Motorstator angeordnet ist, zu erkennen. Ferner befindet sich stromabwärts des Motors ein magnetischer Ring 82'. Innerhalb des Fluidkanals 40' befindet sich der Rotor 50', welcher im vorliegenden Beispiel zylinderförmig ausgebildet ist und einen mit dem Motor interagierenden Motorrotor, einen magnetischen Ring 68' und einen Flusskonzentrator 70' aufweist. Die magnetischen Ringe 68' und 82' sind in axialer Richtung, d.h. in z-Richtung, magnetisiert. Hiervon unterschieden werden kann die magnetische Orientierung des Motorrotors, welcher im Wesentlichen quer zur z-Richtung magnetisiert ist. Diese Orientierung ist deutlich besser geeignet um durch den Motor in Rotation versetzt zu werden. Ferner sind magnetische Potentiallinien erkennbar, welche jeweils eine bestimmte Magnetfeldstärke repräsentieren

Das stromabwärts des Flusskonzentrators 70' wirkende Magnetfeld wird durch die Sensoranordnung 100, welche beispielsweise Halleffektsensoren 102 und 104 umfasst, registriert. Die Halleffektsensoren sind derart angeordnet, dass die z-Komponente des Magnetfelds gemessen wird. Die beiden Sensoren 102 und 104 sind um die Kanalachse 42'symmetrisch angeordnet. Der Abstand kann dabei beispielsweise zwischen 2 und 15 mm entlang der x-Richtung betragen, je nach Pumpenmaß. Alternativ oder zusätzlich kann ein weiterer Magnetfeldsensor 105, vorzugsweise entlang der Kanalachse 42' vorhanden sein. Der Magnetfeldsensor ist dergestalt, dass auch die Richtung eines Magnetfeldvektors erfasst werden kann, beispielsweise mittels eines GMR Sensors.

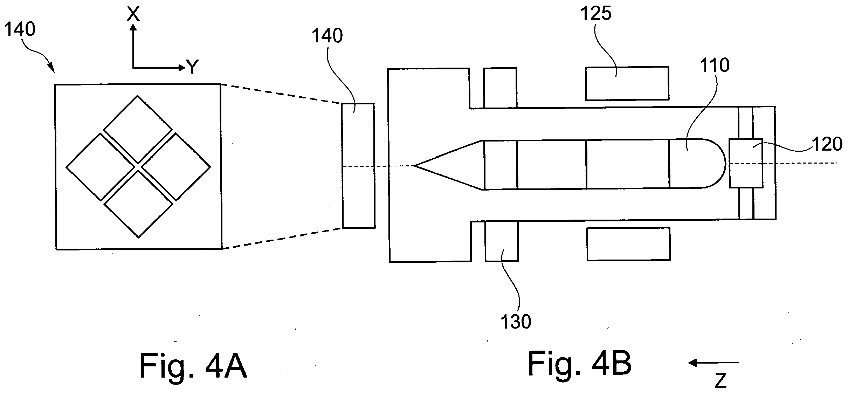

Eine schematische Anordnung der Sensoren ist beispielsweise in den Figuren 4 dargestellt. In der Figur 4a ist ein System aus vier Halleffektsensoren illustriert, welche innerhalb der xy-Achse um den Mittelpunkt auf der z-Achse herum ausgerichtet sind. Dabei sind jeweils zwei entlang der x- bzw. y-Achse ausgerichtete Sensoren in der Figur 4a in der jeweils gleichen Farbe gehalten, um zu illustrieren, dass diese zwei Sensoren miteinander gekoppelt ein differenzielles Messen, und somit eine hohe Messgenauigkeit, ermöglichen. Die Sensoranordnung entlang der z-Richtung ist in der Figur 4b dargestellt. Schematisch ist auch der Rotor 110 zu sehen, welcher an einer mechanischen Barriere 120 punktförmig aufliegt. Der Rotor 110 wird durch den Motor 125 in Rotation versetzt. Das stromabwärtige Ende des Rotors 110 ist durch ein magnetisches Lager 130 radial gelagert. Im Rotor selbst sind Magneten angeordnet, welche derart orientiert sind, dass eine besonders große Magnetfeldkomponente in z-Richtung vorherrscht. Diese kann durch die Sensoranordnung 140 ausgewertet werden. Das stromabwärtige Ende des Rotors 110 ist axial von der Sensoranordnung 140 beabstandet. Die Sensoranordnung 140 selbst befindet sich nicht im Fluidkanal, sondern innerhalb des Gehäuses. Wie in der Figur 4A erkennbar, umfasst die Sensoranordnung vier um die Kanalachse symmetrisch angeordnete Halleffektsensoren. Dabei können jeweils zwei sich entlang der Kanalachse gegenüberliegende Sensoren zu einem Sensorpaar zusammengefasst werden. Auf diese Weise kann der Raumwinkel der Verkippung des Rotors ermittelt werden.

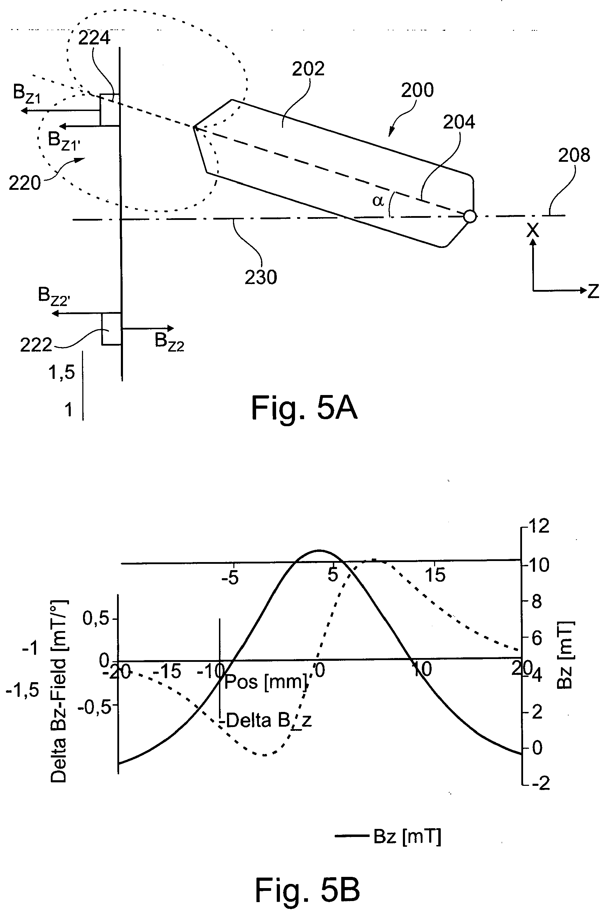

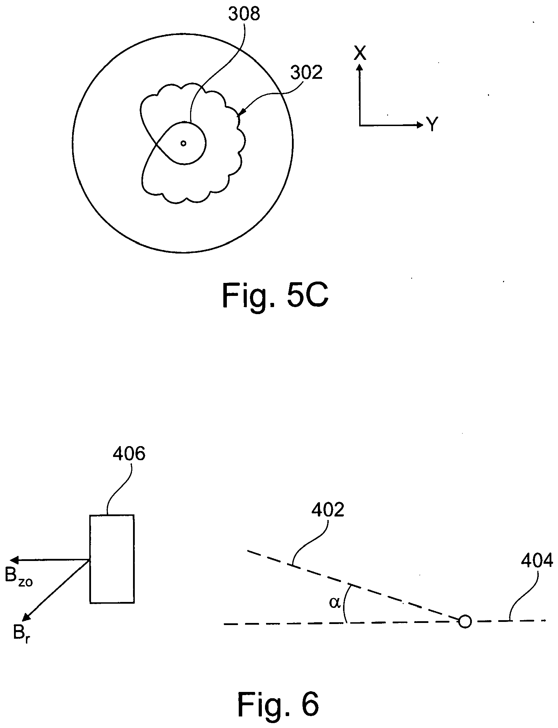

Anhand der Figuren 5 soll auf die Auswertung der durch die Sensoranordnung erfassten Magnetfeldänderungen eingegangen werden. In der Figur 5A ist schematisch ein Rotor 200 dargestellt, welcher einen Rotorkörper 202 mit einer Rotationsachse 204 besitzt. Der Rotor besitzt zudem ein Kugelsegment 206, welches mit einem nicht dargestellten Kalottensegment ein mechanisches Auflager bildet. Der Mittelpunkt der Kugel 206 ist der Schnittpunkt zwischen der Rotationsachse 204 und einer Kanalachse 208, welche das feste Koordinatensystem definiert. In z-Richtung vom Rotor 200 durch die Sensoranordnung befindet sich eine Sensoranordnung 220 mit einem ersten Sensor 222 und einem zweiten Sensor 224. Obgleich hier nur zwei Sensoren dargestellt sind, können selbstverständlich auch mehr, beispielsweise drei oder vier Sensoren, eingesetzt werden. Im dargestellten Zustand ist die Rotationsachse 204 gegenüber der Kanalachse 208 um einen Winkel α verkippt. Die zwei Sensoren 222 und 224 nehmen im nicht-verkippten Zustand des Rotors - aufgrund der Radialsymmetrie des durch den Rotor erzeugten Magnetfelds 230 - identische oder zumindest ähnliche Werte des Magnetfelds des Rotors wahr. Dies ist beispielsweise anhand der Figur 5B verständlich. Die dort dargestellte durchgezogene Kurve des Magnetfelds Bz entspricht dem durch den Rotor erzeugten Magnetfeld Bz entlang der x-Achse im nicht-verkippten Zustand.. Sofern die Magnete nicht ein radial symmetrisches Magnetfeld erzeugen, wird die Kurve der Bz Komponente etwas von der dargestellten Kurve abweichen. Diese Abweichung kann jedoch im Rahmen eines Kalibrierverfahrens in der Auswertung berücksichtigt werden. Wird nun der Rotor verkippt, wie in der Figur 5A angedeutet, nimmt einer der beiden Sensoren ein deutlich größeres Magnetfeld wahr, wohingegen der andere Sensor ein deutlich kleineres Magnetfeld wahrnimmt. Dies ist schematisch dargestellt, indem das Magnetfeld B_{Z1} größer und in seiner Richtung umgekehrt ist als das B_{Z2}. Im Vergleich hierzu ist zu erkennen, dass die gemessenen Magnetfelder im nicht-verkippten Zustand B_{Z1'} und B_{Z2'} im Wesentlichen gleich groß sind.

Die Sensoren werden folgend der Figur 5B mit einem Abstand zur Rotationsachse platziert um den Effekt zu verstärken. Die in Figur 5B gestrichelten Linien liefern die optimale Position der Sensoren entlang der x-Achse für die vorgeschlagene Pumpengeometrie bei etwa 5 mm. Zwei entlang der x-Achse, sich bezüglich der Kanalachse 208 gegenüberliegende Sensoren mit diesem Abstand liefern als differenzielles Sensorpaar im angegebenen Beispiel etwas mehr als 2 mT /° Rotorverkippung als Auflösung. Das Optimum ist von der Pumpengeometrie abhängig. Die differenzielle Messung funktioniert jedoch für beliebige Abstände von der Symmetrieachse.
In der Figur 5A ist lediglich eine Momentaufnahme dargestellt, jedoch ändern sich der Winkel und die Richtung der Verkippung des Rotors 200 über die Zeit. Wird beispielsweise der Rotor durch den Motor in Rotation versetzt und ein Fluid gefördert, wird in Abhängigkeit vom erzeugten Fluss der Rotor um einen Winkel α verkippt werden. Dieser Winkel α muss jedoch nicht konstant sein, sondern kann beispielsweise in einem Zeitintervall in der xy-Ebene wandern. Ferner kann eine in der xy-Ebene beobachtbare Bewegung der Rotationsachse mit einer höherfrequenten Nutationsbewegung überlagert sein.

Hinsichtlich der Auswertung der Magnetfeldänderungen und wie die Verkippung zur Schätzung eines Fluss verwendet werden kann, kann beispielsweise der WO2017/015 268 A1, der WO2009/132 707 A1 oder auch Boening et al, "Evolution of hydraulic radial forces on the impeller by the volute in a centrifugal rotary lock pump", Artificial Organ, Vol. 35, Nr. 8, 2011, entnommen werden.

In der Figur 5c ist eine Trajektorie der stromabwärtigen Spitze des Rotors 200 bzw. der Schnittpunkt der Rotationsachse in einer xy-Ebene dargestellt. Die Trajektorie 300 ist eine geschlossene Trajektorie, was beispielsweise darauf hinweisen kann, dass die aufgrund des Flusses wirkenden Bewegungen harmonische oder subharmonische Beziehungen zur Rotationsfrequenz des Rotors besitzen. Überlagert werden kann diese Trajektorie von einer Nutationsbewegung 302, welche höherfrequent ist als die Frequenz der harmonischen und subharmonischen Schwingungen. Die Nutationsbewegung kann auch zur Erfassung anderer Effekte, wie Verschleißeffekte, oder Ablagerungen, wie Thromben, dienen. Das über eine Zeit ausgewertete Frequenzspektrum der Bewegung der Rotationsachse in der xy-Ebene kann somit verwendet werden um den Zustand der Pumpe abzuschätzen. Auf diese Weise kann zum einen der Fluss abgeschätzt, ein Druck abgeschätzt, die Fluidviskosität abgeschätzt, Ansaugungen detektiert, wachsende und eingespülte Thromben detektiert oder Verschleißerscheinungen an den Lagern oder dem Motor oder sonstige Ablagerungen erkannt werden. Sämtliche Veränderungen des Gleichgewichts zwischen positiver Steifigkeit des Magnetlagers und negativer Steifigkeit zwischen Motorstator und Motormagneten können beobachtet werden. Diese Veränderungen zeigen sich in einer Verschiebung der Resonanzfrequenz sowie der Gleichgewichtsposition. Verschleißeffekte können zum Beispiel am Magnetlagerstator auftreten, wenn dieser durch den Motorstator zu stark erwärmt wird.

Eine weitere Ausführungsform zum Erfassen der Verkippung des Rotors ist schematisch in der Figur 6 dargestellt. Hier sind lediglich die Rotationsachse 402, die Kanalachse 404 und der zwischen diesen vorhandene Winkel α dargestellt. Beim Sensor 406, welcher axial vom Rotor beabstandet ist, handelt es sich um einen Magnetfeldsensor, welcher die Richtung des Magnetfeldvektors im Raum erfassen kann. Im nicht-verkippten Zustand, bei welchem die Rotations- und die Kanalachse koaxial zueinander liegen, misst der Sensor 406 die einen Magnetfeldvektor B_{z}, welcher im Idealfall lediglich entlang der z-Achse ausgerichtet ist. Bei einer Verkippung des Rotors um den Winkel α, misst der Sensor nun einen Magnetfeldvektor Bᵣ, welcher neben einer z-, auch eine x- und/oder y-Magnetfeldkomponente besitzt. Durch die Auswertung der Vektorrichtung kann die Verkippung des Rotors als Raumwinkel erfasst werden.

Auf diese Weise kann mittels eines einzigen Sensors, beispielsweise eines anisotropen magneto-resistiven Sensors (AMR, GMR oder TMR), eine Abschätzung der Verkippung und somit eine Abschätzung über dem durch die Pumpe geförderten Fluss gemacht werden. Mehrere Sensoren für differentielles Messen oder Redundanz sind möglich und verbessern die Messsicherheit bei ansteigender Komplexität.

Weitere Ausführungsbeispiele ergeben sich für den Fachmann in naheliegender Art und Weise.

## Patentansprüche

1. Pumpe, vorzugsweise eine Blutpumpe, umfassend:
- ein Gehäuse mit einem stromaufwärtigen Einlass und einem stromabwärtigen Auslass und einem zwischen dem Einlass und dem Auslass angeordneter Fluidkanal mit einer Kanalachse;
- einen Rotor mit einem stromaufwärtigen und einem stromabwärtigen Ende, wobei der Rotor im Fluidkanal angeordnet ist, eine Beschaufelung zur Förderung des Fluids aufweist, und stromaufwärts durch ein mechanisches oder hydrodynamisches Lager gelagert ist;
- einen im Gehäuse angeordneten Motor, so dass der Rotor um eine Rotationsachse in Rotation versetzt werden kann;
- ein passives magnetisches Lager; und
- eine Sensoranordnung zur Erfassung einer Neigung der Rotationsachse des Rotors.

2. Pumpe nach einem der vorhergehenden Ansprüche, wobei die Sensoranordnung stromabwärts des Rotors angeordnet ist.

3. Pumpe nach einem der vorhergehenden Ansprüche, wobei die Sensoranordnung derart angeordnet oder ausgebildet ist, dass ein in Richtung der Kanalachse verlaufendes Magnetfeld des Rotors messbar ist.

4. Pumpe nach einem der vorhergehenden Ansprüche, wobei die Sensoranordnung mindestens einen magneto-resistiven Sensor oder einen Halleffektsensor aufweist.

5. Pumpe nach einem der vorhergehenden Ansprüche, wobei die Sensoranordnung mindestens drei, vorzugsweise vier oder mehr Sensoren zur Erfassung eines Magnetfelds umfasst.

6. Pumpe nach Anspruch 5, wobei die Sensoranordnung zum differentiellen Messen des Magnetfelds ausgebildet ist.

7. Pumpe nach einem der vorhergehenden Ansprüche, wobei das passiv magnetische Lager ein Radiallager ist.

8. Pumpe nach einem der vorhergehenden Ansprüche, wobei der Rotor einen magnetischen Flusskonzentrator umfasst, welcher vorzugsweise im Bereich des stromabwärtigen Endes des Rotors angeordnet ist.

9. Pumpe nach Anspruch 8, wobei der Rotor stromaufwärts des Flusskonzentrators einen Flussgenerator aufweist, wobei der Flussgenerator vorzugsweise einen Teil des passiven Magnetlagers bildet.

10. Pumpe nach einem der vorhergehenden Ansprüche, wobei der Rotor sich zu seinem stromabwärtigen Ende hin verjüngt.

11. Pumpe nach einem der vorhergehenden Ansprüche, wobei am Gehäuse ein mechanisches Fanglager für das stromabwärtige Endes des Rotors angeordnet ist.

12. Pumpe nach einem der vorhergehenden Ansprüche, wobei der Fluidkanal in eine Auslasskammer übergeht und eine Auslassachse des Auslasses gegenüber der Kanalachse um einen Winkel geneigt ist, vorzugsweise im Wesentlichen senkrecht dazu verläuft.

13. Pumpe nach einem der vorhergehenden Ansprüche, wobei die Pumpe eine Axialpumpe ist oder die Pumpe zur axialen Förderung des Fluids ausgebildet ist oder die Beschaufelung des Rotors wendelförmig ausgebildet ist.

14. Pumpe nach einem der vorhergehenden Ansprüche, wobei die Sensoranordnung mit einer Auswerteeinheit verbunden ist, welche die Neigung des Rotors, vorzugsweise zeitaufgelöst, auswertet.

15. Pumpe nach einem der vorhergehenden Ansprüche, wobei die Sensoranordnung eine Neigung der Rotationsachse gegenüber der Kanalachse mit einer Genauigkeit von 0.05°, vorzugsweise von 0.02°, besonders vorzugsweise von 0.01° oder weniger auflöst.

16. Verwendung einer Pumpe nach einem der vorhergehenden Ansprüche zur Schätzung eines Flusses des Fluids, zur Ermittlung der Viskosität des Fluids, zur Detektion von Ablagerungen im Fluidkanal, zur Messung von Druckdifferenzen und/oder zur Messung eines Verschleißes eines Lagers und/oder anderer Bauteile einer Blutpumpe.

17. Verwendung nach Anspruch 16, wobei die Bewegung des Rotors in mindestens einem Freiheitsgrad eingeschränkt ist.
